# EUROPEAN PATENT APPLICATION

(11) **EP 2 647 374 A1**
(43) Date of publication of application: **09.10.2013**
(21) Application number: 12382138.1
(22) Date of filing: 04.04.2012
(51) Int. Cl.: A61K 31/19, A61P 3/10, A61P 25/00, A61P 37/04, A61P 15/08

(54) **Nutritional compositions including beta-hydroxy-beta-methylbutyrate for regulating transcription factors**

(71) Applicant: Abbott Laboratories, Inc., Abbott Park, IL 60064 (US)
(72) Inventor: López Pedrosa, Jose Maria, 18190 GRANADA (ES); Rueda Cabrera, Ricardo, 18008 GRANADA (ES); Manzano Martin, Manuel Cristobal, 18151 GRANADA (ES)
(74) Representative: Ungria López, Javier

(57) **Abstract**

Disclosed are nutritional compositions that include beta-hydroxy-beta-methylbutyrate. The nutritional compositions may be administered to prevent and/or reduce and/or treat diseases and conditions associated with downregulation of serine-threonine kinase protein kinase B (Akt/PKB) signaling pathways, with the concomitant FOXO transcription factors activation, such as muscle wasting, diabetes, infertility, neurodegeneration, and immune system dysfunction.

## Description

### FIELD OF THE DISCLOSURE

The present disclosure relates to nutritional compositions that include beta-hydroxy-beta-methylbutyrate, and particularly calcium beta-hydroxy-beta-methylbutyrate, for regulating serine-threonine kinase protein kinase B (Akt/PKB) signaling transcription factors. More particularly, the present disclosure relates to regulating forkhead box O (FOXO) transcription factors to prevent and/or reduce and/or treat diseases and conditions associated with upregulation of the FOXO transcription factors.

### BACKGROUND OF THE DISCLOSURE

Muscle atrophy occurs systemically in fasting and a variety of diseases including cancer, diabetes mellitus, acquired immune deficiency syndrome (AIDS), sepsis, and chronic obstructive pulmonary disease (COPD), and in some muscles, upon disuse. In these conditions, muscles show increased rates of protein degradation primarily through activation of the ubiquitin-proteasome pathway. In addition, muscular atrophy is associated with an increase in circulating glucocorticoid levels, suggesting that this hormone may be a trigger for muscular atrophy. A primary action of glucocorticoid in muscle is to inhibit the PI3K/Akt signaling pathway, thereby reducing mTOR activation and inducing forkhead box O activity and atrogin-1 transcription. Therefore, stimulation of the PI3/Akt signaling pathway constitutes some of the most promising future therapeutic approaches to prevent glucocorticoid-induced muscle atrophy.

Additionally, low levels of insulin and IGF-1, together with elevated levels of glucocorticoids, trigger the loss of muscle protein after food deprivation and/or in diabetes, and insulin resistance is a characteristic feature of many systemic diseases that appear to contribute to muscle wasting. Glucocorticoids are necessary for the activation of proteolysis in fasting, diabetes, and sepsis, and in large doses by themselves cause muscle wasting.

IGF-1 and insulin further promote net protein accumulation (i.e., hypertrophy) of mature myotubes and adult muscle fibers. In skeletal muscle, the binding of IGF-1 or insulin activates two major signalling pathways: the Ras-Raf-MEK-ERK pathway and the PI3K/Akt pathway discussed above.

Based on the foregoing, one downstream target of the PI3K/Akt pathway that could mediate these glucocorticoid and/or IGF-1 effects is the FOXO class of transcription factors, a subfamily of the large group of forkhead transcription factors. Mammalian cells contain three members of this family, FOXO1 (FKHR), FOXO3 (FKHRL1), and FOXO4 (AFX). Akt blocks the function of all three by phosphorylation of three conserved amino acid residues, leading to their sequestration in the cytoplasm and resulting in the repression of FOXO3a transcriptional function. Dephosphorylation of FOXO factors leads to nuclear entry and growth suppression or apoptosis. FOXO gene expression is also tightly regulated. Furthermore, FOXO1 is an atrophy-related gene ("atrogene") that is induced in many, perhaps all, types of muscle atrophy and FOXO3 activation causes transcription of the atrogin-1 promoter and a dramatic decrease in fiber size.

Although previous pharmacological therapies have been utilized to reduce muscle wasting, there is a need for an alternative to pharmacological therapy for treating and/or reducing and/or preventing muscle wasting and its related diseases and disorders, and particularly treating and preventing diabetes mellitus, neurodegenerative conditions, and immune system dysfunction. Nutritional compositions capable of modulating activity of the PI3/Akt pathway, and particularly, of the FOXO transcription factors, as a means for treating and/or reducing and/or preventing muscle wasting and other related conditions, including diabetes mellitus, neurodegenerative conditions, and immune system dysfunction, in individuals of all ages would be particularly beneficial.

### SUMMARY OF THE DISCLOSURE

The present disclosure is generally directed to methods of using nutritional compositions including beta-hydroxy-beta-methylbutyrate (HMB), and particularly, calcium HMB. The nutritional compositions generally include calcium HMB in amounts effective to stimulate the PI3K/Akt signalling pathway, and particularly for down-regulating FOXO transcription factors, thereby providing reduced protein degradation and prevention of muscle wasting. Further, as this signaling pathway shows significant involvement in metabolism, cellular proliferation, stress tolerance and lifespan, the nutritional compositions of the present disclosure may be used to prevent, reduce, and/or treat diseases and/or conditions associated with downregulation of serine-threonine kinase protein kinase B (Akt/PKB) signaling pathways, with the concomitant FOXO transcription factors overexpression and activation, including diabetes, immune system dysfunction, neurodegenerative conditions, and infertility, as well as diseases and conditions associated therewith.

The present disclosure is directed to a method of regulating transcription factors in an individual. The method comprises administering an effective amount of beta-hydroxy-beta-methylbutyrate to the individual.

The present disclosure is also directed to a method of regulating a forkhead transcription factor in an individual. The method comprises administering an effective amount of beta-hydroxy-beta-methylbutyrate to the individual.

The present disclosure is also directed to a method of controlling diabetes in an individual. The method comprises administering to the individual an effective amount of beta-hydroxy-beta-methylbutyrate.

The present disclosure is also directed to a method of increasing fertility of an individual. The method comprises administering to the individual an effective amount of beta-hydroxy-beta-methylbutyrate.

The present disclosure is also directed to a method of reducing neurodegeneration. The method comprises administering to the individual an effective amount of beta-hydroxy-beta-methylbutyrate.

The present disclosure is also directed to a method of reducing immune system dysfunction in an individual. The method comprises administering to the individual an effective amount of beta-hydroxy-beta-methylbutyrate.

The present disclosure is also directed to a method for treating a condition related to upregulation of forkhead transcription factor expression and activity by modulating the phosphorylation of Akt kinase in an individual. The method comprises administering an effective amount of beta-hydroxy-beta-methylbutyrate to the individual.

It has been unexpectedly found that HMB, and in particular calcium HMB, when utilized in nutritional compositions regulates serine-threonine kinase protein kinase B (Akt/PKB) signaling, which is involved in cellular metabolism, cellular proliferation, and apoptosis. More particularly, HMB has been found to down-regulate the forkhead box O (FOXO) class of transcription factors, which are tightly controlled by post-translational modifications, including Akt/PKB-mediated phosphorylation, mitogen-activated protein kinase (ERK ½)-mediated phosphorylation, acetylation, and ubiquitylation. FOXO transcription factors control cell proliferation, cell metabolism and cell survival, and thus, are considered to be an attractive therapeutic target for multiple disorders. For example, FOXO overexpression has been shown to lead to disorders and conditions including muscle wasting, diabetes, neurodegeneration and immune system dysfunction. Further, FOXO overexpression has been shown in instances of infertility in both men and women.

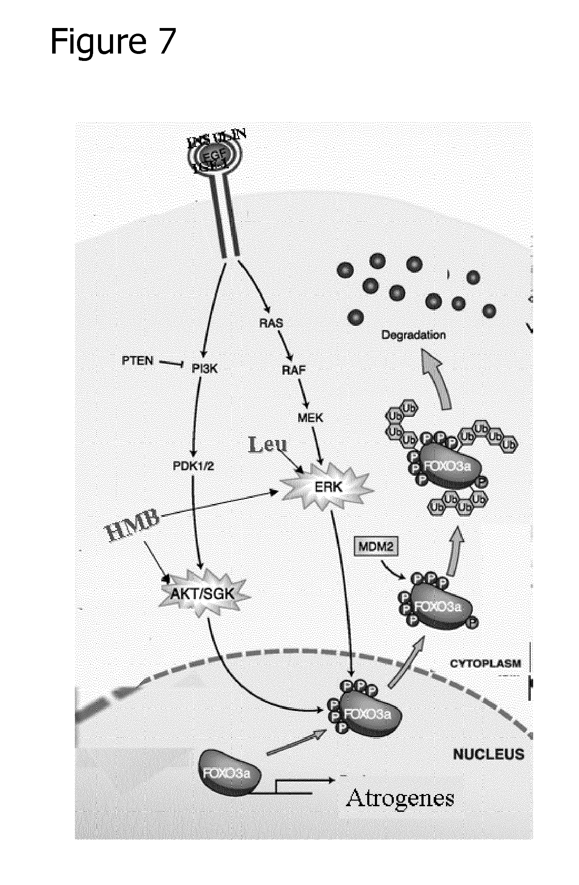

HMB surprisingly shows the capacity to activate both Akt and ERK kinases, which are known to phosphorylate the specific mammalian FOXO transcription factor, FOXO3a, at different phosphorylation sites (see FIG. 7). Akt-dependent phosphorylation of FOXO3a causes FOXO3a nuclear exportation, thereby suppressing FOXO transcriptional activity. Further, FOXO3a phosphorylation by ERK was found to lead to FOXO3a down-regulation via MDM2-mediated proteasome degradation. Accordingly, administration of HMB can prevent and/or reduce and/or treat muscle wasting in catabolic conditions by reducing proteasome-dependent proteolysis and rapidly suppressing the transcription of atrogin-1.

Additionally, it has been discovered that HMB is capable of counteracting the effects of increased circulating glucocorticoid levels, such as found in individuals suffering from muscle atrophy. Particularly, administration of nutritional compositions including HMB reduces protein degradation rate, and thus, may provide significant protein building in the body and a resulting increase in muscle accretion in an individual as compared to individuals administered nutritional compositions without HMB. The HMB may also result in improved glucose tolerance and immune system function in individuals.

### BRIEF DESCRIPTION OF THE DRAWINGS

FIG. 1 is a graph depicting the effects of HMB and Leu on increased protein degradation as analyzed in Example 1.
FIG. 2 is a graph depicting the effects of HMB and Leu on decreased protein synthesis as analyzed in Example 1.
FIG. 3A is a Western Blot and graph depicting the effects of HMB and Leu on the phosphorylation status of PKB/Akt as analyzed in Example 1.
FIG. 3B is a Western Blot and graph depicting the effects of HMB and Leu on the phosphorylation status of mTOR as analyzed in Example 1.
FIG. 3C is a Western Blot and graph depicting the effects of HMB and Leu on the phosphorylation status of atrogin-1 as analyzed in Example 1.
FIG. 4A is a graph depicting the effect of HMB and Leu on FOXO3a transcription factor activity as analyzed in Example 1.
FIG. 4B is a Western Blot and graph depicting the effect of HMB and Leu on FOXO3a transcription factor amount as analyzed in Example 1.
FIG. 5 is a Western Blot and graph depicting the effect of HMB and Leu on the phosphorylation status of ERK ½ as analyzed in Example 1.
FIG. 6 is a graph depicting the effect of HMB and Leu on UbC promoter activity as analyzed in Example 1.
FIG. 7 is a schematic diagram of the PI3K/Akt pathway involved in phosphorylation of FOXO3a transcription factor.

### DETAILED DESCRIPTION OF THE DISCLOSURE

The nutritional compositions of the present disclosure comprise a beta-hydroxy-beta-methylbutyrate that can prevent and/or treat and/or reduce and/or control and/or manage and/or modulate overexpression of FOXO transcription factors, and its related conditions and/or symptoms, and in particular muscle wasting, diabetes, infertility, neurodegeneration and immune system dysfunction, and related diseases and conditions in an individual. These and other optional elements or features of the various embodiments are described in detail hereafter.

These and other features of the nutritional compositions, as well as some of the many optional variations and additions, are described in detail hereafter.

The terms "nutritional composition" or "nutritional product" as used herein, unless otherwise specified, refer to nutritional liquids and nutritional powders, the latter of which may be reconstituted to form a nutritional liquid, all of which comprise one or more of fat, protein, and carbohydrate and are suitable for oral consumption by a human.

The terms "liquid nutritional composition" and "nutritional liquid" are used interchangeably herein, and unless otherwise specified, refer to nutritional products in ready-to-drink liquid form and concentrated form.

The terms "fat" and "oil" as used herein, unless otherwise specified, are used interchangeably to refer to lipid materials derived or processed from plants or animals. These terms also include synthetic lipid materials so long as such synthetic materials are suitable for oral administration to humans.

The term "shelf stable" as used herein, unless otherwise specified, refers to a liquid nutritional composition that remains commercially stable after being packaged and then stored at 18-24°C for at least 3 months, including from about 6 months to about 24 months, and also including from about 12 months to about 18 months.

The terms "neurodegenerative disease" or "neurodegenerative condition" as used herein, unless otherwise specified, are used interchangeably to refer to the progressive loss of structure or function of neurons, including the death of neurons and include diseases such as Parkinson's disease, Alzheimer's disease, Huntington's disease, dementia, amyotrophic lateral sclerosis, stroke, and schizophrenia.

The term "immune system dysfunction" as used herein, unless otherwise specified, refers to the dysfunction and/or underdevelopment of B and T lymphocytes, neutrophils and other non-lymphoid lineases that modulate inflammation in inflammatory-related diseases and conditions such as inflammatory arthritis and systemic lupus erythematosus.

The terms "retort packaging" and "retort sterilizing" are used interchangeably herein, and unless otherwise specified, refer to the common practice of filling a container, most typically a metal can or other similar package, with a liquid nutritional composition and then subjecting the liquid-filled package to the necessary heat sterilization step, to form a sterilized, retort packaged, liquid nutritional product.

The term "aseptic packaging" as used herein, unless otherwise specified, refers to the manufacture of a packaged product without reliance upon the above-described retort packaging step, wherein the liquid nutritional composition and package are sterilized separately prior to filling, and then are combined under sterilized or aseptic processing conditions to form a sterilized, aseptically packaged, liquid nutritional product.

All percentages, parts and ratios as used herein, are by weight of the total composition, unless otherwise specified. All such weights as they pertain to listed ingredients are based on the active level and, therefore, do not include solvents or by-products that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional compositions of the present disclosure may also be substantially free of any optional or selected ingredient or feature described herein, provided that the remaining nutritional composition still contains all of the required ingredients or features as described herein. In this context, and unless otherwise specified, the term "substantially free" means that the selected nutritional composition contains less than a functional amount of the optional ingredient, typically less than 1%, including less than 0.5%, including less than 0.1%, and also including zero percent, by weight of such optional or selected ingredient.

The nutritional compositions may comprise, consist of, or consist essentially of the elements of the nutritional compositions as described herein, as well as any additional or optional element described herein or otherwise useful in nutritional composition applications.

### Product Form

The nutritional compositions used in the methods of the present disclosure include HMB, and in many embodiments calcium HMB, and include ready-to-feed liquids, concentrated liquids, liquids derived from nutritional powders (reconstituted liquids), powders, solids, semi-liquids, and semi-solids. The liquid embodiments may include solutions (including clear solutions), suspensions, and emulsions. The powders that are reconstituted to produce a liquid may include any flowable or scoopable particulate solid that can be diluted with water or other aqueous liquid to form a nutritional liquid prior to use.

The nutritional compositions may be formulated with sufficient kinds and amounts of nutrients to provide a sole, primary, or supplemental source of nutrition, or to provide a specialized nutritional product for use in individuals afflicted with specific diseases or conditions or with a targeted nutritional benefit.

### Nutritional Powders

The nutritional powders may be reconstituted by the intended user with a suitable aqueous liquid, typically water or other aqueous liquid, in an amount or volume sufficient to form a nutritional liquid for immediate oral or enteral use. In this context, "immediate" use generally means within about 48 hours, more typically within about 24 hours, most typically right after or within 20 minutes of reconstitution. Further, when reconstituted, the nutritional powders provide the desired ingredient concentrations as described hereinafter for the nutritional liquid embodiments.

The nutritional powders may include spray dried powders, dry mixed powders, agglomerated powders, combinations thereof, or powders prepared by other suitable methods. In some embodiments, all or a portion of the HMB may be dryblended into the nutritional powder.

### Nutritional Liquids

The nutritional liquids may be formulated in a variety of forms, including emulsions such as oil-in-water, water-in-oil, or complex aqueous emulsions, although such emulsions are most typically in the form of oil-in-water emulsions having a continuous aqueous phase and a discontinuous oil phase, suspensions, or clear or substantially clear liquids.

The nutritional liquids may be and typically are shelf stable. The nutritional liquids typically contain up to 95% by weight of water, including from about 50% to 95%, also including from about 60% to about 90%, and also including from about 70% to about 85%, of water by weight of the nutritional liquid.

The nutritional liquids may have a caloric density tailored to the nutritional needs of the ultimate user, although in most instances the liquids comprise generally at least 19 kcal/fl oz (660 kcal/liter), more typically from about 20 kcal/fl oz (675-680 kcal/liter) to about 25 kcal/fl oz (820 kcal/liter), even more typically from about 20 kcal/fl oz (675-680 kcal/liter) to about 24 kcal/fl oz (800-810 kcal/liter). Generally, the 22-24 kcal/fl oz formulas are more commonly used in preterm or low birth weight infants, and the 20-21 kcal/fl oz (675-680 to 700 kcal/liter) formulas are more often used in term infants. In some embodiments, the liquid may have a caloric density of from about 100 kcal/liter to about 660 kcal/liter, including from about 150 kcal/liter to about 500 kcal/liter.

The nutritional liquid may have a pH ranging from about 3.5 to about 8, but are most advantageously in a range of from about 4.5 to about 7.5, including from about 4.5 to about 7.0, including from about 4.5 to about 6.7, including from about 4.5 to about 6.5, and including from about 4.5 to about 6.0. In some embodiments, the pH range includes from about 5.5 to about 7.3, including from about 5.5 to about 7.0, including from about 5.5 to about 6.5, and including from about 5.5 to about 6.0. In still other embodiments, the pH range may be from about 6.2 to about 7.2, including from about 6.2 to about 7.0, and including from about 6.2 to about 6.5.

Although the serving size for the nutritional liquid can vary depending upon a number of variables, a typical serving size is generally at least 2 mL, or even at least 5 mL, or even at least 10 mL, or even at least 25 mL, including ranges from 2 mL to about 300 mL, including from about 4 mL to about 250 mL, and including from about 10 mL to about 240 mL.

### Beta-Hydroxy-Beta-Methylbutyrate (HMB)

The nutritional compositions used in the methods of the present disclosure include HMB, and preferably calcium HMB, which means that the nutritional compositions are either formulated with the addition of calcium HMB, most typically as a monohydrate, or are otherwise prepared so as to contain HMB in the finished composition. Any source of HMB is suitable for use herein provided that the finished product contains HMB, although such a source is preferably calcium HMB and is most typically added as such to the nutritional compositions during formulation.

Although calcium HMB monohydrate is the generally preferred source of HMB for use in the nutritional compositions disclosed herein, other suitable sources may include HMB as the free acid, a salt, an anhydrous salt, an ester, a lactone, or other product forms that otherwise provide a bioavailable form of HMB from the nutritional compositions. Non-limiting examples of suitable salts of HMB for use herein include HMB salts, hydrated or anhydrous, of sodium, potassium, magnesium, chromium, calcium, or other non-toxic salt form. Calcium HMB monohydrate is preferred and is commercially available from Technical Sourcing International (TSI) of Salt Lake City, Utah.

The concentration of HMB in the nutritional compositions may range up to 20%, including from about 0.01% to about 10%, including from about 0.01% to about 8%, and also including from about 0.08% to about 5.0%, and also including from about 0.08% to about 3%, and also including from about 0.1% to about 2.5%, and also including from about 0.1% to about 2.0%, and also including from about 0.1% to about 0.5%, by weight of the nutritional composition. In some specific embodiments, the nutritional compositions include about 0.38% or about 0.71%, by weight HMB.

The nutritional compositions of the present disclosure desirably include sufficient HMB to provide an individual with from about 0.1 grams to about 10 grams, including from about 0.5 grams to about 10 grams, including from about 1 gram to about 8 grams, including from about 2 grams to about 7 grams, and also including from about 3 grams to about 6 grams, per day of HMB. In one specific embodiment, the daily intake of HMB by the individual is about 3 grams. The total daily HMB may be contained in one, two, three, or more servings of the nutritional composition.

### Macronutrients

The nutritional compositions may further comprise one or more optional macronutrients in addition to the HMB described herein. The optional macronutrients include proteins, fats, carbohydrates, and combinations thereof. The nutritional compositions are desirably formulated as dietary products containing all three macronutrients.

Macronutrients suitable for use herein include any protein, fat, or carbohydrate or source thereof that is known for or otherwise suitable for use in an oral nutritional composition, provided that the optional macronutrient is safe and effective for oral administration and is otherwise compatible with the other ingredients in the nutritional composition.

The concentration or amount of optional fat, carbohydrate, and protein in the nutritional composition can vary considerably depending upon the particular product form (e.g., bars or other solid dosage forms, milk or soy-based liquids or other clear beverages, reconstitutable powders, etc.) and the various other formulations and targeted dietary needs. These optional macronutrients are most typically formulated within any of the embodied ranges described in the following tables.

| **Nutrient (% total calories)** | **Example A** | **Example B** | **Example C** |
|---|---|---|---|
| Carbohydrate | 0-100 | 10-70 | 40-50 |
| Fat | 0-100 | 20-65 | 35-55 |
| Protein | 0-100 | 5-40 | 15-25 |

Each numerical value preceded by the term "about"

| **Nutrient (wt% composition)** | **Example D** | **Example E** | **Example F** |
|---|---|---|---|
| Carbohydrate | 0-98 | 1-50 | 10-30 |
| Fat | 0-98 | 1-30 | 3-15 |
| Protein | 0-98 | 1-30 | 2-10 |

Each numerical value preceded by the term "about"

### Carbohydrate

Optional carbohydrates suitable for use in the nutritional compositions may be simple, complex, or variations or combinations thereof, all of which are optionally in addition to the HMB and calcium HMB as described herein. Non-limiting examples of suitable carbohydrates include hydrolyzed or modified starch or cornstarch, maltodextrin, isomaltulose, sucromalt, glucose polymers, sucrose, corn syrup, corn syrup solids, rice-derived carbohydrate, glucose, fructose, lactose, high fructose corn syrup, honey, sugar alcohols (e.g., maltitol, erythritol, sorbitol), and combinations thereof.

Optional carbohydrates suitable for use herein also include soluble dietary fiber, non-limiting examples of which include gum Arabic, fructooligosaccharide (FOS), sodium carboxymethyl cellulose, guar gum, citrus pectin, low and high methoxy pectin, oat and barley glucans, carrageenan, psyllium and combinations thereof. Insoluble dietary fiber is also suitable as a carbohydrate source herein, non-limiting examples of which include oat hull fiber, pea hull fiber, soy hull fiber, soy cotyledon fiber, sugar beet fiber, cellulose, corn bran, and combinations thereof.

Carbohydrate concentrations in the nutritional compositions, for example, may range from about 0.1% to about 70%, including from about 0.1% to about 50%, including from about 5% to about 70%, including from about 3% to about 65%, including from about 7% to about 30%, and including from about 10% to about 20%, by weight of the nutritional composition. In some embodiments, the carbohydrate is present in an amount of from about 0.1% to about 50%, including from about 0.5% to about 4.0%, including from about 0.5% to about 3.5%, including from about 1.0% to about 3.5%, including from about 1.5% to about 3.5%, including from about 1.5% to about 3.0%, including from about 2.0% to about 2.5%, and including about 2.4%, by weight of the nutritional composition.

### Protein

In addition to the HMB, the nutritional compositions of the present disclosure may further comprise one or more proteins or sources thereof that are suitable for use in oral nutritional compositions and are compatible with the elements and features of such compositions. Total protein concentrations in the nutritional compositions may range from about 0.5% to about 50%, including from about 1% to about 45%, and also including from about 2% to about 40%, and including from about 5% to about 35%, including from about 5% to about 30%, including from about 5% to about 25%, including from about 7% to about 25%, including from about 10% to about 25%, including from about 10% to about 20%, by weight of the nutritional composition. In one specific embodiment, the total protein concentration may be from about 1.0% to about 30% by weight, of the nutritional composition. In other specific embodiments, the total protein concentration may be from about 0.5% to about 25%, including from about 1.0% to about 20%, including from about 1.0% to about 10%, including from about 5% to about 9%, including from about 7% to about 9%, including from about 7.5% to about 8.5%, including from about 7.7% to about 8.2%, including about 8.1% by weight of the nutritional composition.

Optional proteins suitable for use in the nutritional compositions include hydrolyzed, partially hydrolyzed or non-hydrolyzed proteins or protein sources, and can be derived from any known or otherwise suitable source such as milk (e.g., casein, whey), animal (e.g., meat, fish, egg albumen), cereal (e.g., rice, corn), vegetable (e.g., soy, pea, potato), or combinations thereof. The proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-carnitine, and combinations thereof.

### Fat

In addition to the HMB, the nutritional compositions may further comprise fat or sources thereof, most typically as emulsified fat, concentrations of which may range from about 0.1% to about 40%, including from about 1% to about 30%, including from about 1% to about 20%, including from about 1% to about 15%, and also including from about 1% to about 10%, by weight of the nutritional composition. In some embodiments, the fat concentration may be from about 0.1% to about 25%, including from about 0.5% to about 4.0%, including from about 0.5% to about 3.0%, including from about 0.5% to about 2.0%, including from about 0.5% to about 1.5%, including from about 0.5% to about 1.0%, including from about 0.75% to about 1.0%, including from about 0.8% to about 1.0%, including from about 0.85% to about 0.95%, and also including about 0.9% by weight of the nutritional composition.

Suitable sources of fat for use herein include any fat or fat source that is suitable for use in an oral nutritional product and is compatible with the elements and features of such products. Desirably, a fat source will provide at least one long chain polyunsaturated acid (LC-PUFA) such as DHA, ARA, and/or EPA, although these LC-PUFAs may be optionally added to the nutritional compositions outside of, or in addition to, the fat source.

Non-limiting examples of suitable fats or sources thereof for use in the nutritional emulsions described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

### Optional Ingredients

The nutritional compositions described herein may further comprise other optional ingredients that may modify the physical, chemical, hedonic or processing characteristics of the products or serve as pharmaceutical or additional nutritional components when used in the targeted population. Many such optional ingredients are known or otherwise suitable for use in other nutritional compositions and may also be used in the nutritional compositions described herein, provided that such optional ingredients are safe and effective for oral administration and are compatible with the essential and other ingredients in the selected product form.

Non-limiting examples of such optional ingredients include preservatives, antioxidants, emulsifying agents, buffers, pharmaceutical actives, additional nutrients as described herein, colorants, flavors, thickeners (e.g., induced viscosity fibers), additional stabilizers, cereal beta-glucans (barley beta-glucan), probiotics (e.g., *Lactobacillus rhamnosus* HN001 (DR20)), prebiotics (fructooligosaccharides, galactooligosaccharides, inulin, oligofructose), *Salacia* extract, and so forth.

The products may further comprise vitamins or related nutrients, non-limiting examples of which include vitamin A, vitamin D, vitamin E, vitamin K, thiamine, riboflavin, pyridoxine, vitamin B12, carotenoids, niacin, folic acid, pantothenic acid, biotin, vitamin C, choline, inositol, salts, and derivatives thereof, and combinations thereof.

The products may further comprise minerals, non-limiting examples of which include phosphorus, magnesium, calcium, iron, zinc, manganese, copper, sodium, potassium, molybdenum, chromium, selenium, chloride, and combinations thereof.

The products may also include one or more flavoring or masking agents. Suitable flavoring or masking agents include natural and artificial sweeteners, sodium sources such as sodium chloride, and hydrocolloids, and combinations thereof.

### Methods of Manufacture

The HMB-containing nutritional compositions for use in the methods described herein may be manufactured by any known or otherwise suitable method for making the nutritional product form selected. When manufactured as nutritional liquids, the nutritional liquids may either be suitably sterilized by aseptic sterilization or by retort sterilization. Nutritional liquids may be prepared, for example, by any of the well known methods of formulating nutritional liquids by way of retort, aseptic packaging, or hot fill processing methods. Such methods are well known in the nutrition formulation and manufacturing arts.

In one suitable manufacturing process, for example, at least three separate slurries are prepared, including a protein-in-fat (PIF) slurry, a carbohydrate-mineral (CHO-MIN) slurry, and a protein-in-water (PIW) slurry. The PIF slurry is formed by heating and mixing the oil (e.g., canola oil, corn oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents. The resulting CHO-MIN slurry is held for 10 minutes with continued heat and agitation before adding additional minerals (e.g., potassium chloride, magnesium carbonate, potassium iodide, etc.), and/or carbohydrates (e.g., HMOs, fructooligosaccharide, sucrose, corn syrup, etc.). The PIW slurry is then formed by mixing with heat and agitation the remaining protein, if any.

The resulting slurries are then blended together with heated agitation and the pH adjusted to 6.6-7.0, after which the composition is subjected to high-temperature short-time (HTST) processing during which the composition is heat treated, emulsified and homogenized, and then allowed to cool. Water soluble vitamins and ascorbic acid are added, the pH is adjusted to the desired range if necessary, flavors are added, and water is added to achieve the desired total solid level. The composition is then aseptically packaged to form an aseptically packaged nutritional emulsion. This emulsion can then be further diluted, heat-treated, and packaged to form a ready-to-feed or concentrated liquid.

In another embodiment, the nutritional composition is a solid nutritional composition such as a nutritional powder. Any methods known in the nutritional art for preparing nutritional powders may be used herein. By way of example, the nutritional powders can be prepared by drying the heated, homogenized liquid nutritional composition described above, such as by spray drying.

### Methods of Use

The methods of the present disclosure utilize nutritional compositions as described herein that comprise HMB, and in many embodiments, calcium HMB. The nutritional compositions including the HMB can be administered to regulate serine-threonine kinase protein kinase transcription factors (Akt/PKB), and in particular, forkhead box O (FOXO) transcription factors. Surprisingly, nutritional compositions including HMB can be administered to prevent and/or reduce and/or treat and/or manage and/or modulate and/or regulate diseases and conditions resulting from upregulation of FOXO transcription factors, and particularly, upregulation of FOXO1 FOXO3, and FOXO4. Exemplary diseases and conditions that can be addressed with the nutritional compositions including the HMB include diabetes, infertility, neurodegeneration, immune system dysfunction and diseases and conditions associated therewith.

More particularly, HMB has the unexpected capacity to activate both Akt and ERK kinases, which phosphorylate the specific FOXO3a transcription factor at different phosphorylation sites. Akt-dependent phosphorylation of FOXO3a promotes FOXO3a nuclear export to the cytoplasm, resulting in the repression of FOXO3a transcriptional function. ERK-dependent phosphorylation of FOXO3a induces its conformational change and promotes the physical interaction of FOXO3a with the E3 ubiquitin ligase MDMe, leading to subsequent FOXO3a ubiquitination and proteasomal degradation.

Activation of Akt, which leads to dephosphorylation of FOXO transcription factors, is cytoprotective, thereby protecting cells from free radical exposure, hyperglycemia, hypoxia, β-amyloid toxicity and oxidative stress. For example, in diabetes, phosphorylation of FOXO transcription factors decreases the levels of pancreatic-duodenal homeobox factor-1 (PDX1), which results in B-cell dysfunction. Additionally, in the liver, FOXO1 phosphorylation contributes to the pathology of diabetes progression by increasing gluconeogenesis and hepatic glucose output. These benefits may be useful for individuals that are diabetics (or individuals who are at risk of or susceptible to becoming diabetics due to obesity, family history, etc.).

Additionally, HMB shows the capacity to downregulate these transcription factors that are further involved in cellular response to oxidative stress, which has been found to be an important determinant in the apoptotic cascade in spermatozoa, providing a pro-survival effect on spermatozoa and preventing and/or reducing and/or treating male infertility. In women, oxidative stress can also lead to infertility and may play a role in the development of polycystic ovary syndrome. Accordingly, by preventing phosphorylation, and thus, downregulating the activity of these FOXO transcription factors, the nutritional compositions including HMB can be administered to prevent and/or reduce and/or treat diseases and conditions including diabetes and infertility.

Likewise, by regulating oxidative stress, the nutritional compositions can also be utilized to improve a cognitive impairment and/or neurodegeneration that may be associated with a neurodegenerative condition. Specifically, oxidative stress has been shown to be associated with the pathogenesis of neurodegenerative conditions such as Alzheimer's disease, Huntington's disease, Parkinson's disease, dementia, amyotrophic lateral sclerosis, stroke, and/or schizophrenia. By reducing this oxidative stress response, neurodegeneration may be prevented and/or reduced and/or treated.

Additionally, as FOXO transcription factors play an important role in regulating immunological homeostasis and tolerance by controlling the function and development of B and T lymphocytes, neutrophils and other non-lymphoid lineages, the nutritional compositions may be administered to modulate inflammation in disease states such as inflammatory arthritis and systemic lupus erythematosus.

The benefits described in the numerous method embodiments herein may be useful for individuals that actually have the disease or condition (i.e., actually have or are recognized as having diabetes, infertility, neurodegeneration, immune system dysfunction, etc.), or for individuals who may be at risk of, susceptible to, or prone to getting the disease or condition (due to family history, lifestyle, environmental factors, etc.) Based on the foregoing, because some of the method embodiments of the present disclosure utilizing HMB are directed to specific subsets or subclasses of identified individuals (that is, the subset or subclass of individuals "in need" of assistance in addressing one or more specific disease or specific conditions noted herein as described above), not all individuals will fall within the subset or subclass of individuals as described herein for certain diseases or conditions.

To obtain any of these above-described benefits, the HMB-containing nutritional compositions may be utilized on a daily basis for a time period of at least one week, or at least two weeks, or at least three weeks, or at least one month, or at least two months, or at least three months, or at least six months, or at least one year, or continually.

### EXAMPLES

The following examples illustrate specific embodiments and or features of the nutritional compositions of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the composition, unless otherwise specified.

The exemplified compositions are nutritional products prepared in accordance with manufacturing methods well known in the nutrition industry for preparing nutritional compositions.

### Example 1

In this Example, the effects of HMB and its precursor, Leucine (Leu), on protein degradation mediated by activation of the ubiquin proteosome pathway in L6 myotubes treated with dexamethasone was analyzed. Additionally, down-stream targets of the P13/Akt pathway responsible for muscle wasting development were identified.

Dexamethasone-treated myotubes have been used in multiple previous studies as an in vitro model to define mechanisms of muscle wasting. Dexamethasone treatment results in increased protein degradation and upregulated expression of several genes in the ubiquitin-proteasome proteolytic pathway, and these and other metabolic changes induced by dexamethasone resemble changes seen in atrophying muscle in experimental animals.

### Materials and Methods

**Materials.** HMB and leucine, were purchased from Sigma (St. Louis, Missouri). Tissue culture media and supplements were from Sigma and Invitrogen (Carlsbad, California). Fetal Calf Serum (FCS) was obtained from Cultek (Madrid, Spain). FOXO3a, ERK1/2 and phospho-p44/42 MAPK E10 (Thr202/Tyr204), mTOR and phospho- mTOR (Ser 2448), Akt/PKB and phospho-PKB/Akt (Ser473) antibodies were obtained from Cell Signaling (Beverly, Massachusetts). Atrogin-1 antibody was obtained from Acris Antibodies GmbH (Herford, Germany) and Histone H3 antibody was provided by Epitomics (Burlingame, California). The Cignal FOXO Reporter (luc) kit was from SABiosciences (Quiagen Inc., Valencia, California). HRP-conjugated secondary antibodies were from Sigma.

**Cell culture**. The L6.C11 rat skeletal muscle myoblast line (ECACC No. 92102119) was grown in DMEM containing 10% (v/v) FCS, 2 mmol/l Glutamine plus 100 units/ml penicillin and 0.1 mg/ml streptomycin (Sigma) in an atmosphere of air/CO₂ (95:5) and maintained at subconfluent densities in growth media. Cells were differentiated to myotubes by exchanging the growth medium for a differentiation medium consisting of DMEM containing 2% (v/v) FCS for 3-4 days (>50% fusion into multinucleated myotubes).

**Protein synthesis and degradation assays**. For analysis of total protein synthesis, 1 µCi/ml [3H]tyrosine ([3H]Tyr, specific activity 42.6 Ci/mmol; Perkin-Elmer) was added to L6-myotube cultures for 1 hour after a 2 hour pre-incubation period with effectors in the presence or absence of dexamethasone (5µM). At the completion of the labeling period, cultures were rinsed three times with PBS, cells were lysed with sodium desoxycholate 0.1%/NaOH 0.1 M and protein was precipitated with cold 10% TCA in PBS overnight, rinsed once with cold 10% TCA, solubilized in NaOH 0.5 M, neutralized with HCl 0.5 M and mixed with scintillation fluid, and radioactivity was determined using a scintillation counter. For the analysis of protein degradation, myotubes (3 days in differentiation media) were labeled with 1 µCi/ml [3H]Tyr for 48 hours in DMEM plus 10% FBS in the presence of effectors: HMB, Leu, Arginine or citrulline, and incubated for an additional 48 hours with 2 mM unlabeled tyrosine in the presence or absence of dexamethasone plus the amino acids or their metabolites. Degradation rates were determined according to Hong & Forsberg, 1995.

**Protein phosphorylation analysis.** L6 myotubes were incubated with HMB or Leu in the absence or presence of dexamethasone for 30 minutes. After treatment, plates were flash frozen in liquid nitrogen and were scraped with 50 µl/60 mm/plate of cold 30 mmol/L Tris-HCl pH 7.4, 25 mmol/L NaCl, 1% (v/v) Triton X-100, 0.1% SDS, 10 mmol/L sodium fluoride, 10 mmol/L sodium pyrophosphate, 1 mmol/L sodium orthovanadate, 1 mmol/L EGTA, 20 nmol/L okadaic acid, 10 µg/ml aprotinin, 10 µg/ml leupeptin, 10 µg/ml pepstatin. After 10 minutes on ice, extracts were centrifuged at 13000 g for 10 minutes at 4°C. Protein concentration from the supernatants was measured using the Bio-Rad Protein Assay. Proteins (25-40 µg) were separated by SDS-PAGE and immunoblotted with selected antibodies; the immunoblots were developed by chemiluminescence detection.

**Atrogin-1 analysis.** L6 myotubes were pre-incubated with HMB 25 µM or Leu 10 mM for 48 hours. Then, dexamethasone 5 µM was added for another 48 hours. After treatment, cells were lysed with RIPA buffer (Sigma) supplemented with inhibitor protease cocktail (Sigma). Lysates were sonicated and protein concentration was measured using the Bio-Rad Protein Assay. Proteins (40 µg) were separated by SDS-PAGE and immunoblotted with anti-atrogin antibody.

**Reporter gene constructs**. For the analysis of rat Ubiquitin (UbC) promoter, a 417 bp fragment of 417 bp flanking the transcription start point was amplified from rat genomic DNA. This fragment was similar to those that have been characterized for the human and rat promoters. The sequences of all constructs were verified by automated DNA sequencing. The Cignal FOXO Reporter (luc) kit, CCS-1022L, was provided by SABiosciences and contains a mixture of inducible FOXO-responsive firefly luciferase construct and constitutively expressing Renilla luciferase construct (40:1).

**DNA transfection assays.** For transfection experiments, cells were used at 80-90% confluence. Transfection was performed using 4 µl of LipofectAMINE 2000 (Invitrogen) and 1.6 µg of DNA/1 x 10⁵ cells for a 12-well plate in 1 ml of culture medium, following the manufacturer's instructions. The DNA mixture comprised the various pGL3-UbC luciferase reporters and the reference plasmid pRL-TK (ratio 95:5) or the Cignal FOXO Reporter mixture. The pRL-TK plasmid codes for Renilla luciferase under the control of the TK promoter and served as an internal control to correct for differences in transfection efficiencies within an experiment. Cells were exposed to the mixture of LipofectAMINE 2000 and plasmid for 5 hours. Following removal of the transfection reagent, fresh medium was added to the cultures. In the case of cultures set to differentiate into myotubes, differentiation medium was applied 24 hours later and was applied for a total of 3 days. Luciferase activity was determined using standard reagents (Dual Luciferase method, Promega) in a luminometer (Sirius L, Berthold Technologies, Bad Wildbad, Germany) and results were standardized for Renilla luciferase activity. All transfections were performed at least three times, in triplicate, using at least two preparations of plasmid DNA. To allow comparison of the expression patterns, the data were expressed as relative changes in luciferase activity and were normalized to a value of 100%.

**Preparation of nuclear extracts**. Nuclear extracts from L6 cells were prepared following Bhavsar et al. Protein concentration was measured in nuclear extracts using the Bio-Rad Protein Assay kit. Aliquots of the extracts (40 µg) were resolved by 10% SDS-PAGE and analyzed by immunoblotting using a 1:1000 dilution of polyclonal anti-FOXO3a or anti-Histone H3 antibodies. The antigen-antibody complexes were detected with HRP-conjugated secondary antibodies using a chemiluminescence detection system (Bio-Rad).

**Statistical methods.** Results are expressed as mean ± SEM. Statistical analysis was performed by ANOVA followed by Student's t-test as appropriate. P < 0.05 was considered statistically significant.

### Effect of HMB, in comparison to leucine (Leu), on protein degradation and synthesis in dexamethasone treated L6 myotubes

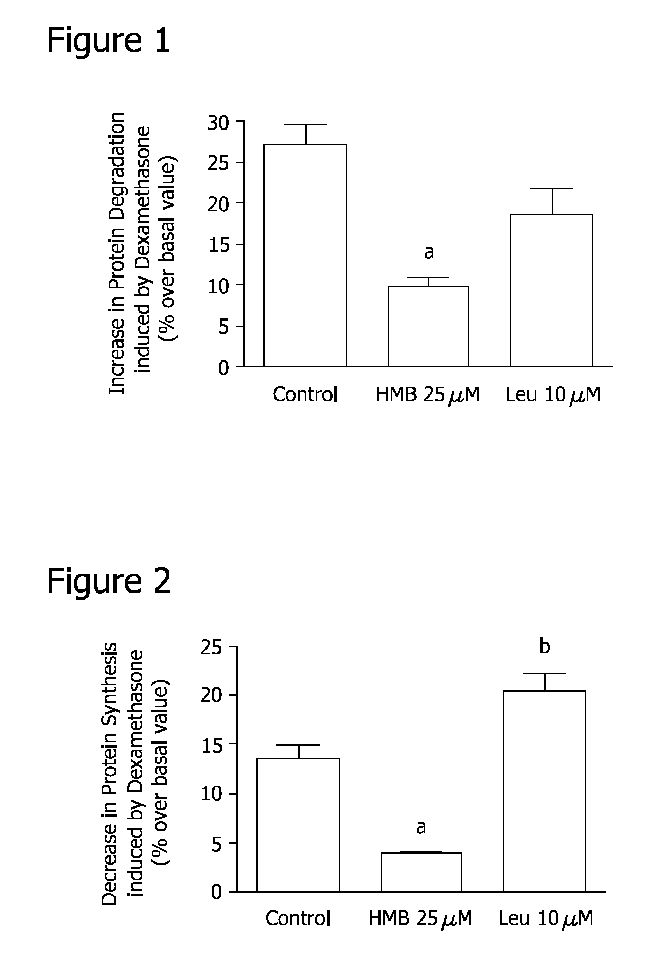

To study the effects of HMB and Leu on protein degradation induced by dexamethasone, L6 differentiated myotubes were first differentiated to myotubes for 3 days in 2% FBS, and then the medium was changed to 10% FBS for the remainder of the experiment. Myotubes were pre-incubated with HMB (25µM) or Leu (10 mM) for 48 hours. After the incubation, medium was removed and supplemented with cold tyrosine to prevent reincorporation of radioactivity to the newly synthetized proteins. Cells were then incubated with effectors for an additional 48 hours in the presence or absence of 5 µM dexamethasone. After the incubation, protein degradation for each condition was calculated. The results are shown in FIG. 1 as the increase in protein degradation (%) due to the presence of dexamethasone when compared with the incubations in the presence of effectors alone. Results are expressed as mean ± SEM of six independent experiments.

As expected, incubation with dexamethasone induced a significant increase on protein degradation (FIG. 1) in L6 myotubes. The results are similar to previous reports in which Dexametasone stimulated protein degradation in L6 myotubes. Pre-treatment of the cultured cells with HMB significantly decreased proteolyis induced by Dexametasone (approximately 60% in protein degradation). However, Leu pre-treatment did not significantly affect glucocorticoid-induced protein degradation in cultured L6 myotubes.

The increase in protein degradation caused by dexamethasone was found to be associated with reduced protein synthesis. Particularly, L6 myotubes were treated with HMB (25 µM) or Leu (10 mM) for 2 hours in the presence or absence of dexamethasone (5 µM). After incubation, medium was removed and supplemented with 3[H]-Tyr in the presence of the effectors and/or dexamethasone for an additional period of one hour. After the incubation, protein synthesis for each condition was calculated. The results are shown in FIG. 2 as the decrease in protein synthesis (%) due to the presence of dexamethasone when compared with the incubations in the presence of effectors alone. Results are expressed as means ± SEM of six independent experiments.

As shown in FIG. 2, in L6 myotubes, dexamethasone induced a significant decrease in protein synthesis. Further, HMB, but not Leu, was able to prevent the decrease in protein synthesis induced by glucocorticoid treatment.

### Effect of HMB, in comparison to Leu, on PI3/Akt pathway in L6 myotubes undergoing atrophy by dexamethasone treatment

To identify the signaling pathways that activate muscle atrophy, changes in the phosphorylation levels and expression of intermediates in the PI3/Akt pathway under various conditions were analyzed. Since glucocorticoid treatment leads to reduction in protein synthesis and increase in protein degradation, the effects of HMB, and its precursor, Leu, on Akt, mTOR phosphorylation levels and atrogin-1 amount were analyzed.

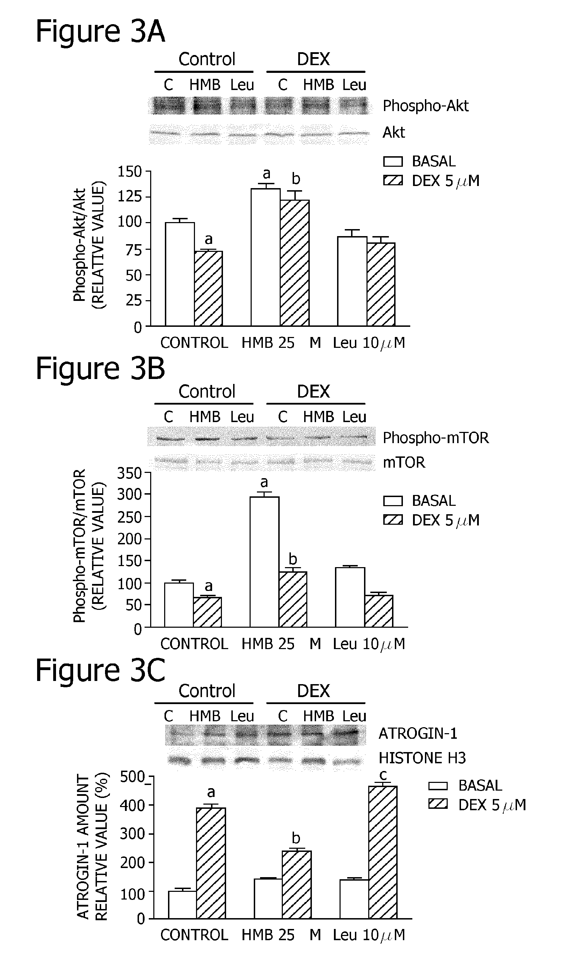

L6 Myoblasts were differentiated to myotubes for 3 days in 2% FBS. Cells were deprived of FBS for 12 hours and incubated with HMB (25 µM) or Leu (10 mM) and insulin (100 nmol/l) in the absence or presence of dexamethasone (5 µM) for 30 minutes. Cells were lysed in phosphorylation buffer and analyzed by Western blot using an anti-phospho PKB/Akt antibody (FIG. 3A), anti-phospho mTOR antibody (FIG. 3B), or anti-atrogin-1 antibody (FIG. 3C). As a loading control, results were normalized using an anti-total PKB/Akt antibody, anti-total mTOR antibody, or an anti-Histone H3 antibody (see FIGS. 3A-3C, respectively). Representative Western blots showing PKB/Akt, mTOR, or Atrogrin-12 and Histone H3 are depicted in FIGS. 3A-3C. The lower panels of each of FIGS. 3A-3C show densitometric quantification of: phospho-PKB/total PKB protein abundance (FIG. 3A); phospho-mTOR/total mTOR protein abundance (FIG. 3B); or atrogin-1 protein abundance using Histone H3 (FIG. 3C) as a reference control. Results are expressed as means ± SEM of four independent experiments.

Dexamethasone treatment significantly reduced the level of phospho-AKT (FIG. 3A), phosphor mTOR (FIG. 3B) and increased more than 4-fold the amount of atrogin-1 (FIG. 3C). The addition of HMB to the culture medium significantly stimulated the phosporylative state of Akt and mTOR in L6 myotubes. When HMB-treated L6 myotubes were exposed to dexamethasone, the phosphorylative state of Akt and mTOR was similar and even higher than those found at basal levels in control untreated cells. In summary, HMB could avoid Akt and mTOR dephosphorylation induced by dexamethasone by increasing the phophorylative levels of these mediators at basal levels.

HMB, alone, did not influence the amount of atrogin-1 in L6 myotubes, but significantly blunted the dexamethasone induced atrogin-1 expression. Leu treatment at basal levels did not have influence on the phosporylative state of Akt and mTOR, and was not able to prevent mTOR dephosphorylation induced by dexamethasone. Furthermore, Leu pre-treatment was not able to block glucocorticoid-induced atrogin-1 expression.

To address whether the down-regulation of atrogin-1 in HMB- pretreated cells was accompanied by an underlying regulation of the FOXO transcription factor, the total FOXO expression (monitored by using FOXO-luciferase reporter gene activity) and the amount FOXO3a transcription factor in the nucleus were analyzed. L6 Myoblasts were differentiated to myotubes for 3 days in 2% FBS. After the differentiation process, medium was changed to 10% FBS for the remainder of the experiment. Myotubes were incubated in the presence or absence of HMB (25 µM) or Leu (10 mM) for 48 hours. After the incubation, dexamethasone (5 µM) was added and further incubated for an additional period of 12 hours. L6 cells transfected with a mixture of a FOXO-responsive luciferase construct and a constitutively expressing Renilla construct (40:1) were treated as above and the luciferase dependent activity was assayed as described in the Material and Methods section.

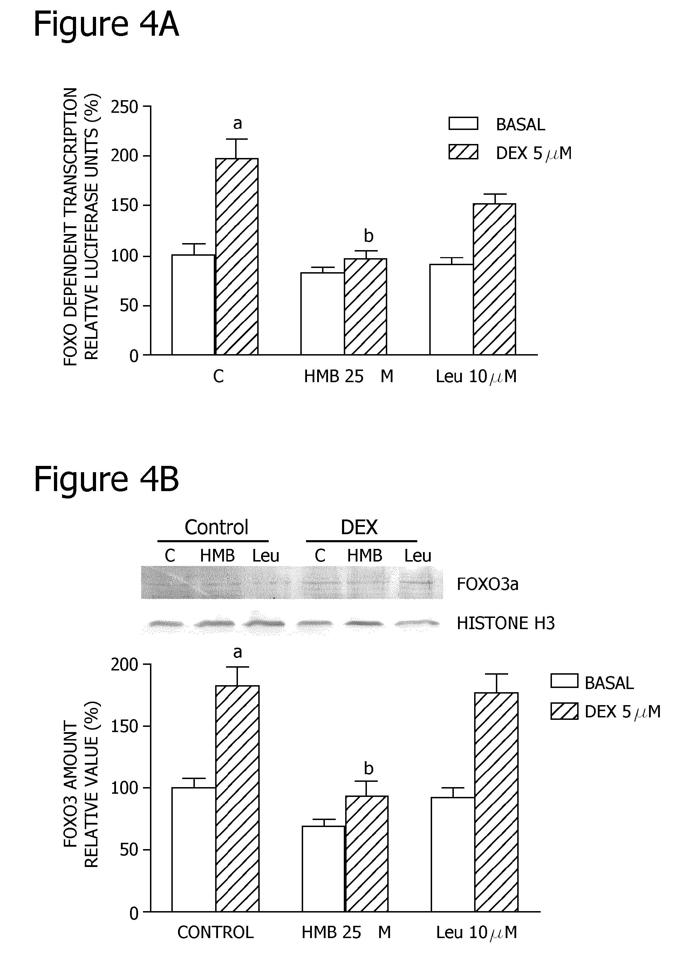

Further, L6 untransfected cells were treated as above, nuclear extracts were obtained as described in the material and methods section and analyzed by Western blot using an anti-FOXO3 antibody. As a loading control, results were normalized using an anti-Histone H3 antibody. Representative Western blots showing FOXO3a and Histone H3 are depicted in FIGS. 4A and 4B. The lower panel of FIG. 4B shows the densitometric quantification of FOXO3 protein abundance using Histone H3 as a reference control. Signal densities from untreated cells were assigned a value of 100%. Results are expressed as means ± SEM of four independent experiments.

Treatment of myotubes with dexamethasone resulted in FOXO cellular overexpression (FIG. 4A) with a parallel increase in FOXO3a transcription factor translocation from the cytoplasm to the nucleus (FIG. 4B). Pre-treatment of myotubes with HMB sharply reduced FOXO overexpression and FOXO3a nuclear translocation induced by dexamethasone. Pre-incubation with Leu did not have any effect on the dexamethasone-increased FOXO expression and amount. Finally, it was shown that under basal conditions, in the absence of dexamethasone, neither HMB nor Leu were able to significantly change FOXO basal activity or amount.

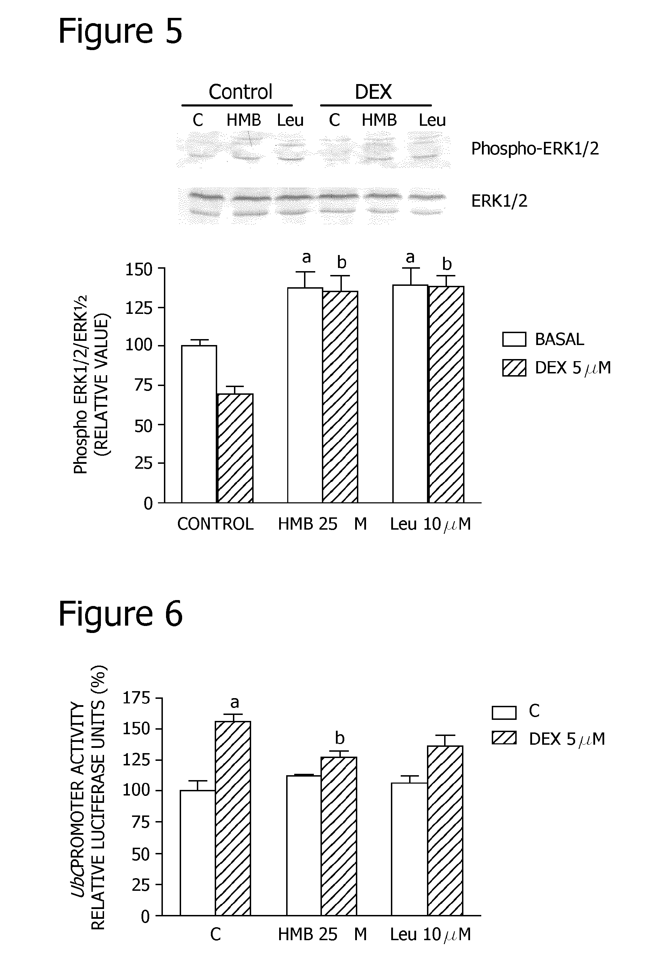

Taking into account that phosphorylation of FOXO3a by ERK is a required step for FOXO3a ubiquitination and subsequent proteosomal degradation of this transcription factor in the cytoplasm, the effect of HMB and Leu on ERK activation was also analyzed. Specifically, L6 Myoblasts were differentiated to myotubes for 3 days in 2% FBS. Cells were deprived of FBS for 12 hours and incubated with HMB (25 µM) or Leu (10 mM) and insulin (100 nmol/l) in the absence or presence of dexamethasone (5 µM) for 30 minutes. Cells were lysed in phosphorylation buffer and analyzed by Western blot using an anti-phospho ERK1/2 antibody. As a loading control, results were normalized using an anti-total ERK1/2 antibody. Representative Western blots showing ERK1/2 are depicted in FIG. 5. The lower panel of FIG. 5 shows the densitometric quantification of phospho-ERK/total ERK protein abundance. Signal densities from untreated cells were assigned a value of 100%. Results are expressed as means ± SEM of four independent experiments.

ERK activation was significantly blunted with the dexamethasone treatment (FIG. 5). However, both effectors, HMB and Leu, induced an activation of ERK kinase either at basal or at atrophic conditions.

Additionally, the effect of HMB and Leu on UbC promoter implicated in protein degradation induced by dexamethasone was analyzed. Particularly, since the UbC mRNA is typically increased more than the other ubiquitin mRNAs (i.e., UbA and UbB) in muscle wasting in catabolic patients, a sensitive marker for analyzing general activation of the Ub-proteosome in atrophying muscles, a reporter system based on the rat Ub-C promoter activity, was used. Specifically, L6 cells transfected with a pGL3-Ubc reporter luciferase construct and a constitutively expressing Renilla construct (95:5) were differentiated to myotubes for 3 days in 2% FBS. After the differentiation process, myotubes were incubated in the presence or absence of HMB (25 µM) or Leu (10 mM) for 48 hours. Then, dexamethasone (5 µM) was added and further incubated for an additional period of 48 hours and luciferase dependent activity was assayed as described in the material and methods section. Results are expressed as means ± SEM of six independent experiments.

As shown in FIG. 6, dexamethasone treatment induced an increase in the UbC promoter activity. Experiments where the cells were pretreated in the presence of HMB (25 µM) or Leu (10 mM) before being challenged with dexamethasone (5 µM), showed that, again, only HMB was able to significantly counteract the effects of dexamethasone on Ub-C promoter activity.

The above results indicate that HMB and Leu exert different muscle wasting-related responses to dexamethasone in L6 myotubes. HMB was able to produce a significant protective effect on the dexamethasone-increased protein degradation rate, while Leu was not able to significantly decrease the dexamethasone-induced degradation. Furthermore, when protein synthesis was assayed with these effectors in the presence of dexamethasone, only HMB was able to recover partly the decrease in protein synthesis caused by dexamethasone treatment.

The results also provide strong evidence that the anti-proteolytic action of HMB is exerted by inactivation of FOXO transcriptional function. FOXO down-regulation induced by HMB, but not by Leu, could be explained based on HMB showing the capacity to activate both Akt and ERK kinases, while Leu only activated ERK kinase. It is known that Akt and ERK phosphorylate FOXO3a at different phosphorylation sites. Akt-dependent phosphorylation of FOXO3a (Thr32, Ser253, and Ser315 for human FOXO3) promotes FOXO3a nuclear export to the cytoplasm, resulting in the repression of FOXO3a transcriptional function. ERK-dependent phosphorylation of FOXO3a (Ser344, and Ser425) induces its conformational change and promotes the physical interaction of FOXO3a with the E3 ubiquitin ligase MDM2, leading to subsequent FOXO3a ubiquitination and proteasomal degradation. The fact that Leu only targeted ERK, but not Akt, may lead to FOXO localization in the nucleus and subsequent transcription of the atrophy-related gene.

These findings together indicate an important new mechanism of HMB contributing to the prevention of muscle wasting in catabolic conditions by reducing proteasome-dependent proteolysis, rapidly suppressing the transcription of atrogin-1, and thus blocking the atrophy program.

### Example 2

Example 2 illustrates a liquid nutritional emulsion of the present disclosure, the ingredients of which are listed in the table below. All ingredient amounts are listed as kg per 1000 kg batch of product, unless otherwise specified.

| Ingredients | Approximate Amount per 1000 Kg |
|---|---|
| Ingredient water | Q.S. |
| Sucrose | 98.2 |
| Whey Protein Isolate | 33.2 |
| Phosphoric Acid, 85% | 1.5 |
| Citric Acid | 1.1 |
| Calcium HMB | 5.31 |
| Flavor | 700 g |
| Ascorbic Acid | 535 g |
| Coloring Agent | 400 g |
| UTM/TM Premix | 230 g |
| Water Dispersible Vitamins A,D,E,K Premix | 178 g |
| Water Soluble Vitamin Premix | 37.9 g |
| Folic Acid | 1.3 g |
| Potassium Iodide | 204 mg |

### Examples 3-7

Examples 3-7 illustrate nutritional powders of the present disclosure including calcium HMB, the ingredients of which are listed in the table below. These products are prepared by spray drying methods in separate batches, are reconstituted with water prior to use to the desired target ingredient concentrations. All ingredient amounts are listed as kg per 1000 kg batch of product, unless otherwise specified.

| **Ingredient** | **Ex. 3** | **Ex. 4** | **Ex. 5** | **Ex. 6** | **Ex. 7** |
|---|---|---|---|---|---|
| Maltodextrin | 436.7 | 436.7 | 436.7 | 436.7 | 436.7 |
| Sucrose | 145.5 | 145.5 | 145.5 | 145.5 | 145.5 |
| Calcium Caseinate | 129.1 | 129.1 | 129.1 | 129.1 | 129.1 |
| Isolated Soy Protein | 57.7 | 57.7 | 57.7 | 61.7 | 57.7 |
| FOS Powder | 33.6 | 33.6 | 33.6 | 33.6 | 32.6 |
| HO sunflower oil | 59.9 | 55.5 | 61.24 | 57.2 | 62.58 |
| Calcium HMB | 31.6 | 34.6 | 28.6 | 27.6 | 32.6 |
| Canola Oil | 55.1 | 53.7 | 56.4 | 52.42 | 57.78 |
| Soy Oil | 26.7 | 26.0 | 27.37 | 25.36 | 28.04 |
| Potassium Citrate | 10.3 | 10.3 | 10.3 | 10.3 | 10.3 |
| Sodium Citrate | 5.8 | 5.8 | 5.8 | 5.8 | 5.8 |
| Potassium Chloride | 5.2 | 5.2 | 5.2 | 5.2 | 5.2 |
| Magnesium Chloride | 4.7 | 4.7 | 4.7 | 4.7 | 4.7 |
| Potassium hydroxide | 3.1 | 3.1 | 3.1 | 3.1 | 3.1 |
| Sodium phosphate dibasic dihydrate | 3.0 | 3.0 | 3.0 | 3.0 | 3.0 |
| Sodium chloride | 2.5 | 2.5 | 2.5 | 2.5 | 2.5 |
| Choline Chloride | 1.8 | 1.8 | 1.8 | 1.8 | 1.8 |
| Flavor | 2.8 | 2.8 | 2.8 | 2.8 | 2.8 |
| Sodium phosphate monobasic monohydrate | 1.6 | 1.6 | 1.6 | 1.6 | 1.6 |
| Potassium phosphate dibasic trihydrate | 1.1 | 1.1 | 1.1 | 1.1 | 1.1 |
| Vitamin premix | 1.065 | 1.065 | 1.065 | 1.065 | 1.065 |
| Ascorbyl palmitate | 0.243 | 0.243 | 0.243 | 0.243 | 0.243 |
| Ascorbic acid | 0.240 | 0.240 | 0.240 | 0.240 | 0.240 |
| Tocopherol-2 antioxidant | 0.116 | 0.116 | 0.116 | 0.116 | 0.116 |
| Ferrous sulfate | 0.010 | 0.090 | 0.030 | 0.020 | 0.070 |
| Zinc sulfate monohydrate | 0.057 | 0.057 | 0.057 | 0.057 | 0.057 |
| Manganese sulfate | 0.045 | 0.045 | 0.045 | 0.045 | 0.045 |
| Mineral mix copper sulfate | 0.035 | 0.035 | 0.035 | 0.035 | 0.035 |
| Beta carotene 30% | 0.005 | 0.005 | 0.005 | 0.005 | 0.005 |
| Chromium chloride | 0.001 | 0.001 | 0.001 | 0.001 | 0.001 |
| Sodium molybdate | 0.0012 | 0.0012 | 0.0012 | 0.0012 | 0.0012 |
| Potassium iodide | 0.001 | 0.001 | 0.001 | 0.001 | 0.001 |
| Sodium selenite | 0.0004 | 0.0004 | 0.0004 | 0.0004 | 0.0004 |
| Citric acid | AN | AN | AN | AN | AN |
| Potassium hydroxide | AN | AN | AN | AN | AN |
| Magnesium sulfate dry | AN | AN | AN | AN | AN |
| Ultra micronized tricalcium phosphate | AN | AN | AN | AN | AN |
| Ascorbic acid | AN | AN | AN | AN | AN |

| | | | | | |
|---|---|---|---|---|---|
| AN = As Needed | | | | | |

### Examples 8-12

Examples 8-12 illustrate nutritional emulsion embodiments of the present disclosure, the ingredients of which are listed in the table below. All amounts are listed as kilogram per 1000 kilogram batch of product, unless otherwise specified.

| **Ingredient** | **Ex. 8** | **Ex. 9** | **Ex. 10** | **Ex. 11** | **Ex. 12** |
|---|---|---|---|---|---|
| Water | Q.S. | Q.S. | Q.S. | Q.S. | Q.S. |
| Sucrose | 89.3 | 89.3 | 89.3 | 89.3 | 89.3 |
| Maltodextrin | 29.7 | 29.7 | 29.7 | 29.7 | 29.7 |
| Sodium Caseinate | 25.9 | 25.9 | 25.9 | 25.9 | 25.9 |
| Milk Protein Caseinate | 19.1 | 19.1 | 19.1 | 19.1 | 19.1 |
| Soy Protein Isolate | 11.9 | 11.9 | 9.9 | 12.9 | 13.9 |
| Potassium Citrate | 7.9 | 7.9 | 7.9 | 7.9 | 7.9 |
| Soy Oil | 11.1 | 9.9 | 11.4 | 10.7 | 11.6 |
| Calcium HMB | 6.7 | 7.7 | 8.7 | 5.7 | 4.7 |
| Canola Oil | 10.2 | 10.0 | 10.5 | 9.8 | 10.7 |
| Corn Oil | 9.3 | 9.1 | 9.6 | 8.9 | 9.8 |
| Whey Protein Concentrate | 3.5 | 3.5 | 3.5 | 3.5 | 3.5 |
| Magnesium Phosphate Dibasic | 3.1 | 3.1 | 3.1 | 3.1 | 3.1 |
| Flavoring Agent | 2.9 | 2.9 | 2.9 | 2.9 | 2.9 |
| Stabilizer | 2.0 | 2.0 | 2.0 | 2.0 | 2.0 |
| Soy Lecithin | 1.5 | 1.5 | 1.5 | 1.5 | 1.5 |
| Sodium Phosphate Dibasic Dihydrate | 1.3 | 1.3 | 1.3 | 1.3 | 1.3 |
| Potassium Phosphate Dibasic | 0.985 | 0.98 5 | 0.985 | 0.985 | 0.985 |
| Potassium Chloride | 0.729 | 0.729 | 0.729 | 0.729 | 0.729 |
| Choline Chloride | 0.480 | 0.480 | 0.480 | 0.480 | 0.480 |
| Ascorbic Acid | 0.469 | 0.469 | 0.469 | 0.469 | 0.469 |
| Calcium Carbonate | 0.451 | 0.451 | 0.451 | 0.451 | 0.451 |
| UTM/TM Premix | 0.367 | 0.367 | 0.367 | 0.367 | 0.367 |
| 45% Potassium Hydroxide | 0.323 | 0.323 | 0.323 | 0.323 | 0.323 |
| Carrageenan | 0.200 | 0.200 | 0.200 | 0.200 | 0.200 |
| Water Soluble Vitamin Premix | 0.185 | 0.185 | 0.185 | 0.185 | 0.185 |
| Vitamin DEK Premix | 0.067 | 0.067 | 0.067 | 0.067 | 0.067 |
| Sodium Chloride | 0.060 | 0.060 | 0.060 | 0.060 | 0.060 |
| Gellan Gum | 0.050 | 0.050 | 0.050 | 0.050 | 0.050 |
| Vitamin A Palmitate in corn oil | 0.008 2 | 0.0082 | 0.0082 | 0.0082 | 0.0082 |
| Corn oil carrier | Q.S. | Q.S. | Q.S. | Q.S. | Q.S. |
| Vitamin D₃ in corn oil | 19 mg | 19 mg | 19 mg | 19 mg | 19 mg |
| Potassium Iodide | 194 mg | 194 mg | 194 mg | 194 mg | 194 mg |

## Claims

1. A method of regulating transcription factors in an individual, the method comprising administering an effective amount of beta-hydroxy-beta-methylbutyrate to the individual.

2. The method of claim 1 wherein the nutritional composition comprises from about 0.1% to about 2.0% beta-hydroxy-beta-methylbutyrate by weight.

3. The method of claim 1 wherein the nutritional composition comprises from about 0.1% to about 0.5% beta-hydroxy-beta-methylbutyrate by weight.

4. The method of claim 1 wherein the beta-hydroxy-beta-methylbutyrate is present as calcium beta-hydroxy-beta-methylbutyrate.

5. The method of claim 1 wherein the nutritional composition is a liquid composition and comprises from about 1% to about 10% protein; from about 10% to about 20% carbohydrate; and from about 1% to about 10% fat, by weight of the liquid composition.

6. A method of regulating a forkhead transcription factor in an individual, the method comprising administering an effective amount of beta-hydroxy-beta-methylbutyrate to the individual.

7. The method of claim 6 wherein the nutritional composition comprises from about 0.1% to about 2.0% beta-hydroxy-beta-methylbutyrate by weight.

8. The method of claim 6 wherein the beta-hydroxy-beta-methylbutyrate is present as calcium beta-hydroxy-beta-methylbutyrate.

9. The method of claim 6 wherein the forkhead transcription factor is selected from the group consisting of FOXO1 (FKHR), FOXO3 (FKHRL1), and FOXO4 (AFX).

10. A method of controlling diabetes in an individual, the method comprising administering to the individual an effective amount of beta-hydroxy-beta-methylbutyrate.

11. The method of claim 10 wherein the nutritional composition comprises from about 0.1% to about 2.0% beta-hydroxy-beta-methylbutyrate by weight.

12. A method of increasing fertility of an individual, the method comprising administering to the individual an effective amount of beta-hydroxy-beta-methylbutyrate.

13. The method of claim 12 wherein the nutritional composition comprises from about 0.1% to about 2.0% beta-hydroxy-beta-methylbutyrate by weight.

14. A method of reducing neurodegeneration in an individual, the method comprising administering to the individual an effective amount of beta-hydroxy-beta-methylbutyrate.

15. The method of claim 14 wherein the nutritional composition comprises from about 0.1% to about 2.0% beta-hydroxy-beta-methylbutyrate by weight.

16. The method of claim 14 wherein the neurodegenerative condition is selected from the group consisting of Parkinson's disease, Alzheimer's disease, Huntington's disease, dementia, amyotrophic lateral sclerosis, stroke, and schizophrenia.

17. A method of reducing immune system dysfunction in an individual, the method comprising administering to the individual an effective amount of beta-hydroxy-beta-methylbutyrate.

18. The method of claim 17 wherein the nutritional composition comprises from about 0.1% to about 2.0% beta-hydroxy-beta-methylbutyrate by weight.

19. The method of claim 17 wherein the immune system dysfunction is an inflammatory disease.

20. A method for treating a condition related to upregulation of forkhead transcription factor expression and activity by modulating the phosphorylation of Akt kinase in an individual, the method comprising administering an effective amount of beta-hydroxy-beta-methylbutyrate to the individual.
